(19) Europäisches Patentamt
European Patent Office
Office européen des brevets

(11) Veröffentlichungsnummer: **0 223 183 A2**

# (12) EUROPÄISCHE PATENTANMELDUNG

(21) Anmeldenummer: **86115593.5**

(22) Anmeldetag: **11.11.86**

(51) Int. Cl.⁴: **C 07 C 127/19**, A 01 N 47/30

(30) Priorität: **22.11.85 DE 3541260**

(43) Veröffentlichungstag der Anmeldung: **27.05.87 Patentblatt 87/22**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Kühle, Engelbert, Dr., von-Bodelschwingh-Strasse 42, D-5060 Bergisch Gladbach 2 (DE)**
Erfinder: **Santel, Hans-Joachim, Dr., Grünstrasse 9a, D-5090 Leverkusen 1 (DE)**
Erfinder: **Schmidt, Robert R., Dr., Im Waldwinkel 110, D-5060 Bergisch Gladbach 2 (DE)**
Erfinder: **Strang, Harry, Dr., Untendorfstrasse 6a, D-4000 Düsseldorf 31 (DE)**

## (54) N-(4-tert.-Butoxy-phenyl)-harnstoffe.

(57) Die Erfindung betrifft N-(4-tert.-Butoxy-phenyl)-harnstoffe der Formel (I),

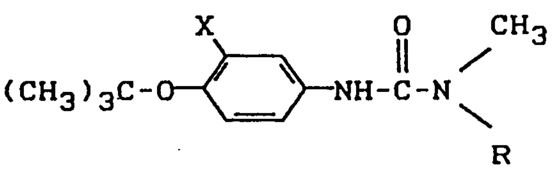

(I)

in welcher
X für Wasserstoff oder Chlor steht und
R für Wasserstoff, Alkyl oder Alkoxy steht,
ein Verfahren zu ihrer Herstellung und ihre Verwendung als Herbizide.

EP 0 223 183 A2

BAYER AKTIENGESELLSCHAFT 5090 Leverkusen, Bayerwerk
Konzernverwaltung RP
Patentabteilung KM/ABc
Ia

# N-(4-tert.-Butoxy-phenyl)-harnstoffe

Die Erfindung betrifft neue N-(4-tert.-Butoxy-phenyl)-harnstoffe, ein Verfahren zu ihrer Herstellung und ihre Verwendung als Herbizide.

Es ist bereits bekannt, daß bestimmte N-Aryl-N'-alkyl-harnstoffe, wie beispielsweise der N-(3-Trifluormethylphenyl)-N',N'-dimethylharnstoff, herbizide Eigenschaften besitzen (vgl. US-P-3 134 665; R. Wegler, Chemie der Pflanzenschutz- und Schädlingsbekämpfungsmittel, Bd. 5, S. 126, Springer Verlag Berlin 1977).

Die herbizide Wirksamkeit dieser vorbekannten Verbindungen gegenüber Unkräuter ist jedoch ebenso wie ihre Verträglichkeit gegenüber wichtigen Nutzpflanzen nicht immer in allen Anwendungsgebieten völlig zufriedenstellend.

Es wurden neue N-(4-tert.-Butoxy-phenyl)-harnstoffe der allgemeinen Formel (I),

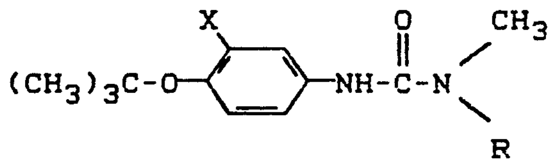

(I)

in welcher

X für Wasserstoff oder Chlor steht und
R für Wasserstoff, Alkyl oder Alkoxy steht,

gefunden.

Weiterhin wurde gefunden, daß man die neuen N-(4-tert.-Butoxy-phenyl)-harnstoffe der Formel (I),

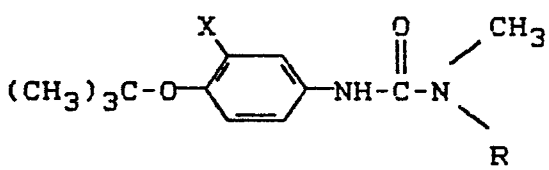

(I)

in welcher

X für Wasserstoff oder Chlor steht und
R für Wasserstoff, Alkyl oder Alkoxy steht,

erhält, wenn man N-(4-tert.-Butoxy-phenyl)-carbamidsäure-O-phenylester der Formel (II),

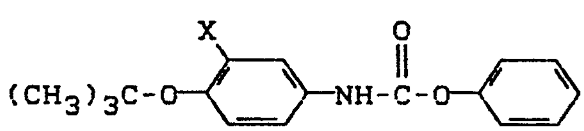

(II)

in welcher

X die oben angegebene Bedeutung hat,

mit Aminen der Formel (III),

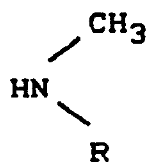

(III)

in welcher

R die oben angegebene Bedeutung hat,

in Gegenwart eines Lösungsmittels umsetzt.

Schließlich wurde gefunden, daß die neuen N-(4-tert.-Butoxy-phenyl)-harnstoffe der Formel (I) herbizide, insbesondere auch selektiv-herbizide Eigenschaften besitzen.

Überraschenderweise zeigen die erfindungsgemäßen N-(4-tert.-Butoxy-phenyl)-harnstoffe der Formel (I) neben einer erheblich verbesserten herbiziden Wirksamkeit gegenüber Problemunkräutern gleichzeitig eine deutlich höhere Verträglichkeit gegenüber wichtigen Kulturpflanzen als die bekannten N-Aryl-N'-alkylharnstoffe, wie beispielsweise der N-(3-Trifluormethylphenyl)-N',N'-dimethylharnstoff, welche chemisch und wirkungsmäßig naheliegende Verbindungen sind.

Die erfindungsgemäßen N-(4-tert.-Butoxy-phenyl)-harnstoffe sind durch die Formel (I) allgemein definiert.

Bevorzugt sind Verbindungen der Formel (I), bei welchen

X für Wasserstoff oder Chlor steht und

R für Wasserstoff, geradkettiges oder verzweigtes Alkyl mit 1 bis 6 Kohlenstoffatomen oder für geradkettiges oder verzweigtes Alkoxy mit 1 bis 4 Kohlenstoffatomen steht.

Besonders bevorzugt sind Verbindungen der Formel (I), bei welchen

X für Wasserstoff oder Chlor steht und

R für Wasserstoff, Methyl, Ethyl, n- oder i-Propyl, n-, i-, s- oder t-Butyl, Methoxy oder Ethoxy steht.

Verwendet man beispielsweise N-(3-Chlor-4-tert.-butoxyphenyl)-O-phenylcarbamidsäureester und Dimethylamin als Ausgangsstoffe, so läßt sich der Reaktionsablauf des erfindungsgemäßen Verfahrens durch das folgende Formelschema darstellen:

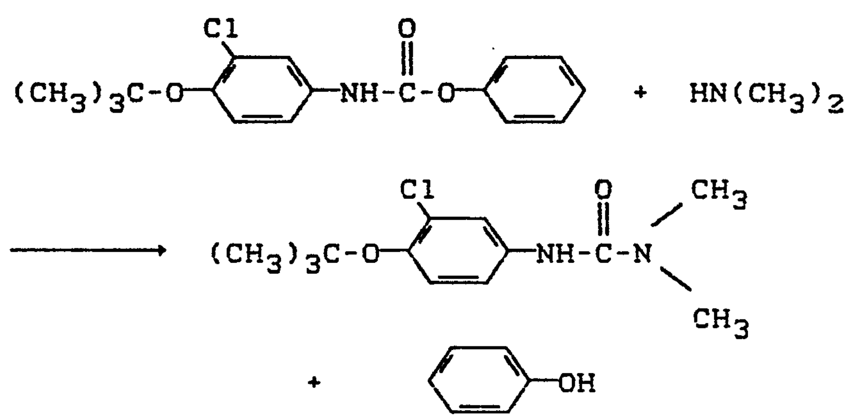

Die zur Durchführung des erfindungsgemäßen Verfahrens als Ausgangsstoffe benötigten N-(4-tert.-Butoxyphenyl)-carbamidsäure-O-phenylester sind durch die Formel (II) allgemein definiert. In der Formel (II) steht X für Wasserstoff oder Chlor.

Die N-(4-tert.-Butoxy-phenyl)-carbamidsäure-O-phenylester lassen sich nach bekannten Verfahren in einfacher Weise herstellen, indem man Anilin-Derivate der Formel (IV),

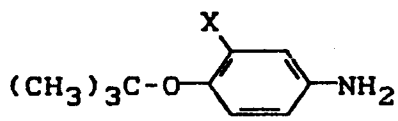

(IV)

in welcher

X für Wasserstoff oder Chlor steht,

mit Chlorameisensäurephenylester der Formel (V)

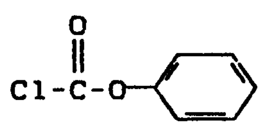

(V)

in Gegenwart eines Verdünnungsmittels wie beispielsweise Toluol und in Gegenwart eines Säurebindemittels wie beispielsweise Triethylamin bei Temperaturen zwischen 10 und 80° C umsetzt (vgl. Houben-Weyl, Methoden der organischen Chemie, Bd. E4, S. 149f, G. Thieme Verlag, Stuttgart (1983).

Die Anilin-Derivate der Formel (IV) sind bekannt oder lassen sich nach bekannten Verfahren herstellen (vgl. J. Chem. Soc. 1954, 1795).

Der Chlorameisensäurephenylester der Formel (V) ist eine allgemein bekannte Verbindung der organischen Chemie.

Die zur Durchführung des erfindungsgemäßen Verfahrens weiterhin als Ausgangsstoffe benötigten Amine sind durch die Formel (III) allgemein definiert. In der Formel (III) steht R vorzugsweise für Wasserstoff, für geradkettiges oder verzweigtes Alkyl mit 1 bis 6 Kohlenstoffatomen oder für geradkettiges oder verzweigtes Alkoxy mit 1 bis 4 Kohlenstoffatomen.

Die Amine der Formel (III) sind allgemein bekannte Verbindungen der organischen Chemie.

Als Ausgangsstoffe der Formel (III) sind ganz besonders geeignet: Methylamin, Dimethylamin, Methylethylamin, Methylbutylamin und O,N-Dimethylhydroxylamin.

Als Verdünnungsmittel zur Durchführung des erfindungsgemäßen Verfahrens kommen inerte organische Lösungsmittel in Frage. Hierzu gehören insbesondere Alkohole wie Methanol oder Ethanol, Kohlenwasserstoffe wie Toluol, chlorierte Kohlenwasserstoffe wie Chloroform und Chlorbenzol oder Ether wie Diethylether, Dioxan und Tetrahydrofuran.

Man kann das erfindungsgemäße Verfahren auch in wäßriger Suspension durchführen, bevorzugt sind Alkohol/Wasser-Gemische.

0223183

Die Reaktionstemperaturen können bei der Durchführung des erfindungsgemäßen Verfahrens innerhalb eines größeren Bereichs variiert werden. Im allgemeinen arbeitet man bei Temperaturen zwischen +10° C und +80° C, vorzugsweise bei Temperaturen zwischen +20° C und +50° C.

Zur Durchführung des erfindungsgemäßen Verfahrens setzt man pro Mol N-(4-tert.-Butoxy-phenyl)-carbamidsäure-O-phenylester der Formel (II) im allgemeinen 1,0 bis 3,0 Mol, vorzugsweise 1,0 bis 2,0 Mol an Amin der Formel (III) ein.

Die Reaktionsdurchführung, Aufarbeitung und Isolierung der Reaktionsprodukte der Formel (I) erfolgen nach üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter in mono- und dikotylen Kulturen, wie beispielsweise Weizen, Mais, Reis und Baumwolle, einsetzen, wobei die Anwendung der erfindungsgemäßen Wirkstoffe mit besonderem Vorzug im Nachauflauf-Verfahren erfolgt.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 4-Amino-6-(1,1-dimethylethyl)-3-ethylthio-1,2,4-triazin-5(5H)-on oder N-(2-Benzthiazolyl)-N,N'-dimethyl-harnstoff zur Unkrautbekämpfung in Getreide; 4Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur

Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,1 und 10 kg Wirkstoff pro Hektar Bodenfläche, vorzugsweise zwischen 0,1 und 5 kg pro ha.

Die Herstellung und die Verwendung der erfindungsgemäßen Wirkstoffe geht aus den nachfolgenden Beispielen hervor.

Herstellungsbeispiele

Beispiel 1

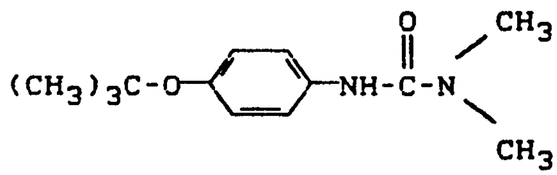

7 g (0,025 Mol) 4-tert.-Butoxyphenylcarbamidsäure-O-phenylester werden in 100 ml Methanol gelöst und mit 1,35 g (0,03 Mol) Dimethylamin versetzt. Hierbei steigt die Temperatur leicht an. Man erhitzt die Reaktionsmischung für 30 Minuten auf 65° C, kühlt ab mit Eiswasser und setzt in der Kälte ca. 20 ml Wasser zu bis zur beginnenden Kristallisation des Reaktionsproduktes.

Man erhält 5 g (86 % der Theorie) an N-4-tert.-Butoxy-phenyl-N',N'-dimethyl-harnstoff von Schmelzpunkt 149-151° C.

Beispiel 2

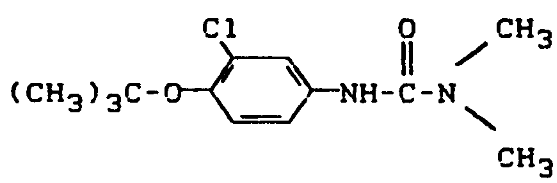

10 g (0,031 Mol) N-(3-Chlor-4-tert.-butoxyphenyl)-carbamidsäure-O-phenylester werden in 100 ml Methanol gelöst

und mit 1,8 g (0,04 Mol) Dimethylamin versetzt. Hierbei steigt die Temperatur leicht an. Man erhitzt eine Stunde zum Sieden, kühlt mit Eiswasser ab und setzt in der Kälte ca. 20 ml Wasser zu bis zur beginnenden Kristallisation.

Man erhält 6 g (83 % der Theorie) an N-(3-Chlor-4-tert.-butoxy-phenyl)-N',N'-dimethyl-harnstoff vom Schmelzpunkt 150-152° C.

In analoger Weise erhält man:

Beispiel 3

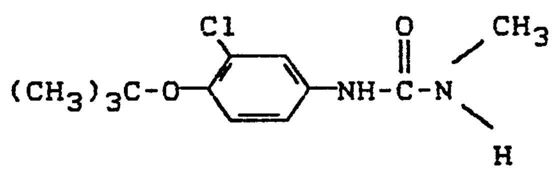

Schmelzpunkt 146-148° C

Beispiel 4

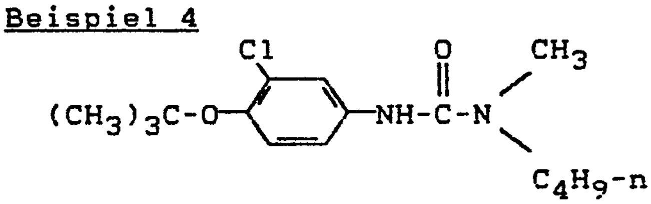

Schmelzpunkt 110-112° C

Beispiel 5

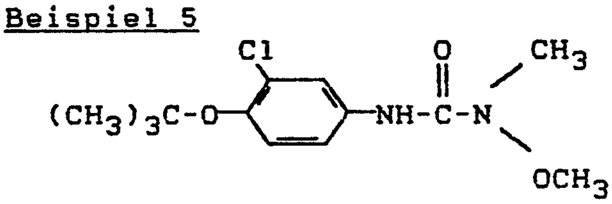

Brechungsindex $n_D^{20}$ = 1,5526

Herstellung der Ausgangsverbindungen

Beispiel II-1

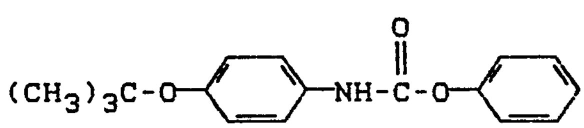

Zu einer Lösung von 7,8 g (0,05 Mol) Chlorameisensäure-O-phenylester in 80 ml Toluol tropft man eine Lösung von 8,2 g (0,05 Mol) 4-tert.-Butoxyanilin ($Kp_{0,1}$: 75 - 80°C) und 6 g (0,05 Mol) Triethylamin in 20 ml Toluol ein. Hierbei steigt die Temperatur bis 58°C an. Nach dem Abkühlen wird die Reaktionsmischung abgesaugt, mit Wasser und anschließend mit Petrolether gewaschen.

Man erhält 11 g (77 % der Theorie) an 4-tert.-Butoxyphenylcarbamidsäure-O-phenylester vom Schmelzpunkt 142-144°C.

In analoger Weise erhält man <u>Beispiel II-2</u>:

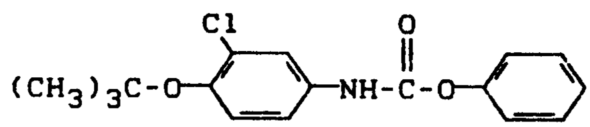

Öl

Anwendungsbeispiele:

In den Anwendungsbeispielen wurde die nachsthend aufgeführte Verbindung als Vergleichssubstanz eingesetzt:

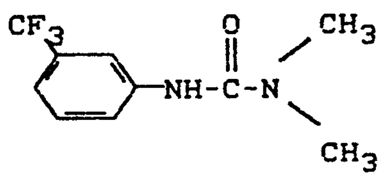

(A)

N-(3-Trifluormethylphenyl)-N',N'-dimethyl-harnstoff (bekannt aus US-P-3 134 665).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität gegenüber der Vergleichssubstanz (A) zeigt in diesem Test z.B. die Verbindung gemäß dem Herstellungsbeispiel 2.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit gegenüber der Vergleichssubstanz (A) zeigt in diesem Test z.B. die Verbindung gemäß dem Herstellungsbeispiel 2.

## Patentansprüche

1. N-(4-tert.-Butoxy-phenyl)-harnstoffe der allgemeinen Formel (I),

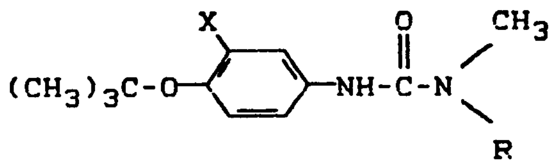

(I)

in welcher

X für Wasserstoff oder Chlor steht und
R für Wasserstoff, Alkyl oder Alkoxy steht.

2. N-(4-tert.-Butoxy-phenyl)-harnstoffe der Formel (I) gemäß Anspruch 1, in welcher

X für Wasserstoff oder Chlor steht und

R für Wasserstoff, für geradkettiges oder verzweigtes Alkyl mit 1 bis 6 Kohlenstoffatomen oder geradkettiges oder verzweigtes Alkoxy mit 1 bis 4 Kohlenstoffatomen.

3. N-(4-tert.-Butoxy-phenyl)-harnstoffe der Formel (I) gemäß Anspruch 1, in welcher

X für Wasserstoff oder Chlor steht und

R für Wasserstoff, Methyl, Ethyl, n- oder i-Propyl, n-, i-, s- oder t-Butyl, Methoxy oder Ethoxy steht.

4. Verfahren zur Herstellung von N-(4-tert.-Butoxyphenyl)-harnstoffen der Formel (I),

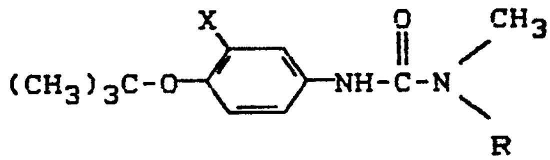

(I)

in welcher

X für Wasserstoff oder Chlor steht und
R für Wasserstoff, Alkyl oder Alkoxy steht,

dadurch gekennzeichnet, daß man N-(4-tert.-Butoxyphenyl)-carbamidsäure-O-phenylester der Formel (II),

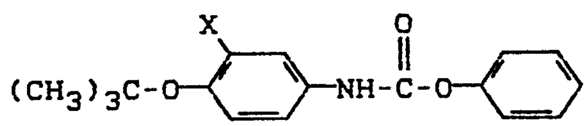

(II)

in welcher

X die oben angegebene Bedeutung hat,

mit Aminen der Formel (III),

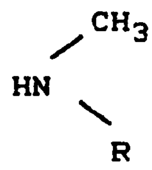

(III)

in welcher

R die oben angegebene Bedeutung hat,

in Gegenwart eines Lösungsmittels umsetzt.

5. Herbizide Mittel, gekennzeichnet durch einen Gehalt an mindestens einem N-(4-tert.-Butoxy-phenyl)-harnstoff der Formel (I) gemäß den Ansprüchen 1 bis 4.

6. Verfahren zur Bekämpfung von Unkräutern, dadurch gekennzeichnet, daß man N-(4-tert.-Butoxy-phenyl)-harnstoffe der Formel (I) gemäß den Ansprüchen 1 bis 4 auf die Unkräuter und/oder deren Lebensraum einwirken läßt.

7. Verwendung von N-(4-tert.-Butoxy-phenyl)-harnstoffen der Formel (I) gemäß den Ansprüchen 1 bis 4 zur Bekämpfung von Unkräutern.

8. Verfahren zur Herstellung von herbiziden Mitteln, dadurch gekennzeichnet, daß man N-(4-tert.-Butoxy-phenyl)-harnstoffe der Formel (I) gemäß den Ansprüchen 1 bis 4 mit Streckmitteln und/oder oberflächenaktiven Substanzen vermischt.